# EUROPEAN PATENT SPECIFICATION

(11) **EP 1 545 453 B1**
(45) Date of publication and mention of the grant of the patent: **18.11.2009**
(21) Application number: 03749288.1
(22) Date of filing: 29.08.2003
(51) Int. Cl.: A61K 9/00, A61K 31/538, A61K 45/06, A61P 31/04, A61K 31/4164

(54) **RIFALAZIL FOR TREATING INFECTIONS OF CLOSTRIDIUM DIFFICILE**
RIFALAZIL ZUR BEHANDLUNG VON INFEKTIONEN MIT CLOSTRIDIUM DIFFICILE
RIFALAZIL POUR LE TRAITEMENT D'INFECTIONS DU CLOSTRIDIUM DIFFICILE

(30) Priority: 29.08.2002 US 406873 P; 03.02.2003 US 444570 P
(43) Date of publication of application: 29.06.2005
(73) Proprietor: ActivBiotics Pharma LLC, Atlanta, GA 30305 (US)
(72) Inventor: EISENSTEIN, Barry, Chestnut Hill, MA 02467 (US)
(74) Representative: Harrison Goddard Foote
(86) International application number: PCT/US2003/027305
(87) International publication number: WO 2004/019907

(56) References cited:
- WO-A-01/53330
- WO-A-03/101445
- US-A- 4 983 602
- US-A- 5 266 315
- US-A- 5 773 000
- US-B1- 6 316 433
- ROTHSTEIN DAVID M; HARTMAN ARTHUR D; CYNAMON MICHAEL H; EISENSTEIN BARRY: "Development potential of rifalazil." EXPERT OPINION ON INVESTIGATIONAL DRUGS., vol. 12, no. 2, February 2003 (2003-02), pages 255-271, XP002340551
- DATABASE BIOSIS BIOSCIENCES INFORMATION SERVICE, PHILADELPHIA, PA, US; AN: PREV200300571826 & DIGESTIVE DISEASE WEEK ABSTRACTS AND ITINARY PLANNER, May 2003 (2003-05),
- DATABASE EMBASE ELSEVIER SCIENCE PUBLISHERS, AMSTERDAM, NL; AN: EMB-2003076290 & EXPERT OPINION ON INVESTIGATIONAL DRUGS, vol. 12, no. 1, 1 February 2003 (2003-02-01), pages 255-271,

## Description

### Background of the Invention

The invention relates to the field of bacterial infections.

*Clostridium* (*C*.) *difficile*, a Gram-positive anaerobic bacterium, is recognized as the major causative agent of colitis (inflammation of the colon) and antibiotic-associated diarrhea that may occur following antibiotic intake. *C. difficile* infection represents one of the most common hospital (nosocomial) infections around the world. In the United States alone, it causes approximately three million cases of diarrhea and colitis per year. This bacterium is primarily acquired in hospitals and chronic care facilities following antibiotic therapy, and is the most frequent cause of outbreaks of diarrhea in hospitalized patients. One of the main characteristics of *C. difficile-*associated colitis is severe inflammation in the colonic mucosa associated with destruction of colonocytes.

The disease initially involves alterations of the beneficial bacteria, which are normally found in the colon, by antibiotic therapy. The alterations lead to colonization by *C. difficile* when this bacterium or its spores are present in the environment. In hospitals or nursing home facilities where *C. difficile* is prevalent and patients frequently receive antibiotics, *C. difficile* infection is very common. In contrast, individuals treated with antibiotics as outpatients have a much smaller risk of developing *C. difficile* infection.

Laboratory studies show that when *C. difficile* colonize the intestinal tract, they release two potent toxins, toxin A and toxin B, which bind to certain receptors in the lining of the colon and ultimately cause diarrhea and inflammation of the large intestine, or colon (colitis). Thus, the toxins are involved in the pathogenesis, or development of the disease.

Although *C. difficile* infection usually responds well to treatment with metronidazole or vancomycin, approximately 15 to 20% of patients will experience re-appearance of diarrhea and other symptoms weeks or even months after initial therapy has been discontinued. The usual therapy for relapse is to repeat the 10 to 14 day course of either metronidazole or vancomycin. A subset of patients continues to relapse whenever antibiotics are discontinued and this represents a therapeutic challenge.

Thus, there is a need for improved methods for treating *C. difficile* infections.

### Summary of the Invention

The invention features the use of rifalazil for the preparation of a pharmaceutical composition for treating an infection of *Clostridium difficile* or for preventing an infection of *Clostridium difficile* in a subject, wherein rifalazil is to be administered to said subject in an effective amount. The dosage of rifalazil can range from 0.01 mg to 1000 mg. The dosage of rifalazil is normally 1 to 1000 mg (desirably 1 to 100 mg, more desirably 1 to 50 mg, and even more desirably 1 to 25 mg). The rifalazil may be given daily (e.g., once, twice, three times, or four times daily) or less frequently (e.g., once every other day, once or twice weekly, or monthly). Rifalazil is administered for a length of time sufficient to treat the subject. Treatment may be for 1 to 31 days, desirably 1 to 21 days, 1 to 14 days or even 1, 3, 5, or 7 days. If desired, treatment can continue for up to a year or even for the lifetime of the subject. In one example, rifalazil is administered at an initial dose of between 5 and 100 mg, followed by subsequent doses of between I and 50 mg for 3 to 7 days. A single dose of rifalazil (e.g., in a dosage of between 1 and 100 mg) can also be employed in the use of the invention. The rifalazil may be administered orally, intravenously, subcutaneously, or rectally.

The use of the invention may be employed as an initial treatment of a subject having or being at risk for developing antibiotic-associated bacterial diarrhea or an infection of *C. difficile*, or it may be employed to treat subjects for whom the initial treatment (e.g., with metronidazole, vancomycin, rifampicin, rifabutin, rifapentine, and rifaximin) has failed to fully treat the antibiotic-associated bacterial diarrhea or an infection of *C. difficile*. The use may be employed, for example, when the subject is colonized with *C. difficile* organisms that are resistant to one or more of metronidazole, vancomycin, rifampicin, rifabutin, rifapentine, and rifaximin.

If desired, rifalazil can be administered with one or more additional antibiotics or with an agent that binds toxin A or toxin B (e.g., the non-absorbed toxin binding polymer GT160-246; U.S. Patent No. 6,270,755). Preferred examples of additional antibiotics are metronidazole, gentamicin, daptomycin, azithromycin, quinupristin, dalfopristin, linezolid, teicoplanin, ciprofloxacin, and vancomycin.

The invention describes administering rifalazil and vancomycin simultaneously or sequentially. Rifalazil and vancomycin can be administered within fourteen days of each other, or within five days, three days, or within twenty-four hours of each other. If desired, either rifalazil or vancomycin, or both can be administered orally. Possible dosages for vancomycin can range from 20 to 2000 mg per day, preferably from 125 to 2000 mg per day, most preferably from 500 to 2000 mg per day.

The invention also describes a medicament for treating a subject having antibiotic-associated bacterial diarrhea or an infection of *Clostridium (C.) difficile,* or preventing the disease or infection in the subject. The medicament comprises rifalazil and vancomycin, which can be suitable for oral or intravenous administration. The unit dosages for rifalazil can range from 0.01 to 100 mg (e.g., between 1 and 100 mg, or between 1 and 50 mg, or between 1 and 25 mg, or between 1 and 5 mg), and the unit dosages for vancomycin can range from 125 to 2000 mg, preferably from 500 to 2000 mg.

The invention also describes a pharmaceutical pack comprising (i) rifalazil in an amount effective to treat a subject having antibiotic-associated bacterial diarrhea or an infection of *C. difficile*; and (ii) instructions for administering the rifalazil to a subject for treating or preventing a *C. difficile* infection. Desirably, the rifalazil is in unit amounts of between 0.01 and 1000 mg (e.g., between 1 and 100 mg, or between 1 and 50 mg, or between 1 and 25 mg, or between 1 and 5 mg), and is present in amounts sufficient to treat for at least 1, 3, 5, 7, 10, 14, 21, or 31 days.

The pharmaceutical pack can further comprise one or more antibiotics. Examples of the additional antibiotic include metronidazole, gentamicin, daptomycin, azithromycin, quinupristin, dalfopristin, linezolid, teicoplanin, ciprofloxacin., and vancomycin. Typical dosages for vancomycin range from 20 to 2000 mg, preferably from 125 to 2000 mg.

Exemplary additional antibiotics that can be administered according to the uses of the invention or included in the above pharmaceutical pack are β-lactams such as penicillins (e.g., penicillin G, penicillin V, methicillin, oxacillin, cloxacillin, dicloxacillin, nafcillin, ampicillin, amoxicillin, carbenicillin, ticarcillin, mezlocillin, piperacillin, azlocillin, and temocillin), cephalosporins (e.g., cepalothin, cephapirin, cephradine, cephaloridine, cefazolin, cefamandole, cefuroxime, cephalexin, cefprozil, cefaclor, loracarbef, cefoxitin, cefmatozole, cefotaxime, ceftizoxime, ceftriaxone, cefoperazone, ceftazidime, cefixime, cefpodoxime, ceftibuten, cefdinir, cefpirome, cefepime, BAL5788, and BAL9141), carbapenams (e.g., imipenem, ertapenem, and meropenem), and monobactams (e.g., astreonam); β -lactamase inhibitors (e.g., clavulanate, sulbactam, and tazobactam); aminoglycosides (e.g., streptomycin, neomycin, kanamycin, paromycin, gentamicin, tobramycin, amikacin, netilmicin, spectinomycin, sisomicin, dibekalin, and isepamicin); tetracyclines (e.g., tetracycline, chlortetracycline, demeclocycline, minocycline, oxytetracycline, methacycline, and doxycycline); lipopetides (e.g., daptomycin); macrolides (e.g., erythromycin, azithromycin, and clarithromycin); ketolides (e.g., telithromycin, ABT-773); lincosamides (e.g., lincomycin and clindamycin); glycopeptides (e.g., vancomycin, oritavancin, dalbavancin, and teicoplanin); streptogramins (e.g., quinupristin and dalfopristin); sulphonamides (e.g., sulphanilamide, para-aminobenzoic acid, sulfadiazine, sulfisoxazole, sulfamethoxazole, and sulfathalidine); oxazolidinones (e.g., linezolid); quinolones (e.g., nalidixic acid, oxolinic acid, norfloxacin, perfloxacin, enoxacin, ofloxacin, ciprofloxacin, temafloxacin, lomefloxacin, fleroxacin, grepafloxacin, sparfloxacin, trovafloxacin, clinafloxacin, gatifloxacin, moxifloxacin, gemifloxacin, and sitafloxacin); rifamycins (e.g., rifampicin, rifabutin, rifapentine, and rifaximin); metronidazole; garenoxacin; ramoplanin; faropenem; polymyxin; tigecycline, AZD2563; and trimethoprim.

By "an effective amount" is meant the amount of rifalazil or rifalazil in combination with one or more additional antibiotics required to result in the *C. difficile* being eradicated from the subject, or to prevent an infection of *C. difficile*, as determined by a diagnostic test that detects *C. difficile*. One example of a diagnostic test is the use of a commercially available enzyme-linked immunoassay (ELISA; Immunocard; Meridian Diagnostics, Inc., Cincinnati, Ohio) to detect the presence of *C. difficile* toxin A protein in cecal content extracts. Another example of a diagnostic test is the use of a cytotoxicity assay using human fibroblast cells (Toxi-Titer; Bartels, Inc., Issaquah, WA) to detect the presence of *C. difficile* toxin B. Both of these examples can be found in McVay and Rolfe (Antimicrobial Agents and Chemo. 44:2254-2258, 2000). An "effective amount" can also mean the amount of rifalazil or rifalazil in combination with one or more additional antibiotics required to reduce the symptoms of the *C. difficile*-associated disease in a subject or animal model. Symptoms include diarrhea, weight loss, lethargy, and ruffled fur in specific animal models. Standard assays present in the art can be used to measure the symptoms of disease (for examples of assays see Boon and Beale, Drugs Suppl. 5:57-63, 1985 and McVay and Rolfe, *supra*). An "effective amount" of rifalazil or rifalazil in combination with one or more additional antibiotics reduces the symptoms of *C. difficile*-associated disease in a subject by 20%, preferably, 30% or 40%, more preferably, 50% or 60%, and most preferably, 70%, 80%, 90%, or more, as compared to an untreated subject.

"Rifalazil" means 3'-hydroxy-5'-(4-isobutyl-1-piperazinyl) benzoxazinorifamycin, also known as KRM-1648 or ABI1648. Methods of making rifalazil and microgranulated formulations thereof are described in U.S. Patents 4,983,602 and 5,547,683, respectively.

"Antibiotic-associated bacterial diarrhea" means a condition in which antibiotic therapy disturbs the balance of the microbial flora of the gut, allowing pathogenic organisms such as *C. difficile* to flourish. These organisms cause diarrhea. Antibiotic-associated bacterial diarrhea includes such conditions as *C. difficile* associated diarrhea (CDAD) and pseudomembranous colitis.

"Pseudomembranous colitis," also known as pseudomembranous enterocolitis or enteritis, means the inflammation of the mucous membrane of both small and large intestine with the formation and passage of pseudomembranous material (composed of fibrin, mucous, necrotic epithelial cells and leukocytes) in the stools.

The term "lower gastrointestinal tract" means the lower part of the small intestine (ileum) and the colon.

The term "enteric coating" means a coating surrounding a core, the solubility of the coating being dependent on the pH in such a manner that it substantially prevents the release of a drug in the stomach, but permits the release of the drug at some stage after the formulation has emptied from the stomach. The term "pH-sensitive enteric polymer" means a polymer the solubility of which is dependent on the pH so that it is insoluble in the gastric juice but dissolves at some stage after the formulation has emptied from the stomach.

By "subject" is meant any warm-blooded animal including but not limited to a human, cow, horse, pig, sheep, goat, bird, mouse, rat, dog, cat, monkey, baboon, or the like. It is most preferred that the subject be a human.

### Detailed Description of the Invention

We have discovered that administration of rifalazil alone or in combination with one or more additional antibiotics is effective to treat a subject having antibiotic-associated bacterial diarrhea or an infection of *Clostridium* (*C.*) *difficile*.

The dosage of rifalazil can range from 0.01 to 1000 mg. The dosage of rifalazil is normally 1 to 1000 mg (desirably 1 to 100 mg, more desirably 1 to 50 mg, and even more desirably 1 to 25 mg). The rifalazil may be given daily (e.g., once, twice, three times, or four times daily) or less frequently (e.g., once every other day, once or twice weekly, or monthly). The administration of rifalazil may be by any suitable means that results in an effective amount of the compound reaching the target region. The compound may be contained in any appropriate amount in any suitable carrier substance, and is generally present in an amount of 1-95% by weight of the total weight of the composition. In one embodiment, the composition is provided in a dosage form that is suitable for oral administration, e.g., a tablet, capsule, pill, powder, granulate, suspension, emulsion, solution, or gel. Alternatively, the composition may be formulated for intravenous or rectal administration. An intravenous formulation of rifalazil is described in U.S. Utility Application Serial No. 10/453,155 (filed June 3, 2003). The pharmaceutical composition can generally be formulated according to conventional pharmaceutical practice (see, e.g., Remington: The Science and Practice of Pharmacy (20th ed.), ed. A.R. Gennaro, 2000, Lippincott Williams & Wilkins, Philadelphia, and Encyclopedia of Pharmaceutical Technology, eds. J. Swarbrick and J. C. Boylan, 1988-1999, Marcel Dekker, New York).

Pharmaceutical compositions according to the invention may be formulated to release rifalazil substantially immediately upon administration or at any predetermined time or time period after administration. The latter types of compositions are generally known as controlled release formulations, which include (i) formulations that create a substantially constant concentration of the drug within the body over an extended period of time; (ii) formulations that after a predetermined lag time create a substantially constant concentration of the drug within the body over an extended period of time; (iii) formulations that sustain drug action during a predetermined time period by maintaining a relatively, constant, effective drug level in the body with concomitant minimization of undesirable side effects associated with fluctuations in the plasma level of the active drug substance (sawtooth kinetic pattern); (iv) formulations that localize drug action by, e.g., spatial placement of a controlled release composition adjacent to or in the diseased tissue or organ; and (v) formulations that target drug action by using carriers or chemical derivatives to deliver the drug to a particular target cell type. Controlled release formulations that may be employed in the method of the invention include those described in U.S. Patent Nos. 5,007,790, 5,525,634, 5,582,837, 5,811,388, 5,849,327, 5,962,024, 5,968,554, 5,972,389, 6,319,518, 6,340,475, 6,488,962, and 6,506,407.

### Solid Dosage Forms For Oral Use

Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (e.g., in order to achieve a controlled release formulation) or it may be adapted not to release the active drug substance until after passage of the stomach; this may be achieved by means of an enteric coating (e.g., a pH-sensitive enteric polymer). Desirably, a substantial amount of the drug is released in the lower gastrointestinal tract. The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or a coating based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose. Furthermore, a time delay material such as, for example, glyceryl monostearate or glyceryl distearate, may be employed.

The solid tablet compositions may include a coating adapted to protect the composition from unwanted chemical changes (e.g., chemical degradation prior to the release of the active drug substance). The coating may be applied on the solid dosage form in a similar manner as that described in Encyclopedia of Pharmaceutical Technology, *supra.*

Formulations for oral use may also be presented as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders and granulates may be prepared using the ingredients mentioned above under tablets and capsules in a conventional manner using, e.g., a mixer, a fluid bed apparatus or a spray drying equipment.

### Controlled Release Oral Dosage Forms

Controlled release compositions for oral use may be constructed to release the active drug by controlling the dissolution and/or the diffusion of the active drug substance.

Any of a number of strategies can be pursued in order to obtain controlled release in which the rate of release outweighs the rate of metabolism of the compound in question. In one example, controlled release is obtained by appropriate selection of various formulation parameters and ingredients, including, e.g., various types of controlled release compositions and coatings. Thus, the drug is formulated with appropriate excipients into a pharmaceutical composition that, upon administration, releases the drug in a controlled manner. Examples include single or multiple unit tablet or capsule compositions, oil solutions, suspensions, emulsions, microcapsules, microspheres, nanoparticles, patches, and liposomes.

Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing rifalazil may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Liquids for Oral Administration

Powders, dispersible powders, or granules suitable for preparation of an aqueous suspension by addition of water are convenient dosage forms for oral administration of rifalazil. Formulation as a suspension provides the active ingredient in a mixture with a dispersing or wetting agent, suspending agent, and one or more preservatives. Suitable dispersing or wetting agents are, for example, naturally-occurring phosphatides (e.g., lecithin or condensation products of ethylene oxide with a fatty acid, a long chain aliphatic alcohol, or a partial ester derived from fatty acids) and a hexitol or a hexitol anhydride (e.g., polyoxyethylene stearate, polyoxyethylene sorbitol monooleate, polyoxyethylene sorbitan monooleate). Suitable suspending agents are, for example, sodium carboxymethylcellulose, methylcellulose, sodium alginate.

### Formulations and dosages for combination therapies

Rifalazil can be administered to a subject having antibiotic associated bacterial diarrhea or an infection of *C. difficile* in conjunction with one or more additional antibiotics. Rifalazil can be administered before, during, or after administration of the additional antibiotics, or any combination thereof. If desired, the administration of rifalazil can be continued while the additional antibiotic is being administered.

Exemplary antibiotics that can be administered in the uses of the invention are β-lactams such as penicillins (e.g., penicillin G, penicillin V, methicillin, oxacillin, cloxacillin, dicloxacillin, nafcillin, ampicillin, amoxicillin, carbenicillin, ticarcillin, mezlocillin, piperacillin, azlocillin, and temocillin), cephalosporins (e.g., cepalothin, cephapirin, cephradine, cephaloridine, cefazolin, cefamandole, cefuroxime, cephalexin, cefprozil, cefaclor, loracarbef, cefoxitin, cefmatozole, cefotaxime, ceftizoxime, ceftriaxone, cefoperazone, ceftazidime, cefixime, cefpodoxime, ceftibuten, cefdinir, cefpirome, cefepime, BAL5788, and BAL9141), carbapenams (e.g., imipenem, ertapenem, and meropenem), and monobactams (e.g., astreonam); β -lactamase inhibitors (e.g., clavulanate, sulbactam, and tazobactam); aminoglycosides (e.g., streptomycin, neomycin, kanamycin, paromycin, gentamicin, tobramycin, amikacin, netilmicin, spectinomycin, sisomicin, dibekalin, and isepamicin); tetracyclines (e.g., tetracycline, chlortetracycline, demeclocycline, minocycline, oxytetracycline, methacycline, and doxycycline); lipopetides (e.g., daptomycin); macrolides (e.g., erythromycin, azithromycin, and clarithromycin); ketolides (e.g., telithromycin, ABT-773); lincosamides (e.g., lincomycin and clindamycin); glycopeptides (e.g., vancomycin, oritavancin, dalbavancin, and teicoplanin); streptogramins (e.g., quinupristin and dalfopristin); sulphonamides (e.g., sulphanilamide, para-aminobenzoic acid, sulfadiazine, sulfisoxazole, sulfamethoxazole, and sulfathalidine); oxazolidinones (e.g., linezolid); quinolones (e.g., nalidixic acid, oxolinic acid, norfloxacin, perfloxacin, enoxacin, ofloxacin, ciprofloxacin, temafloxacin, lomefloxacin, fleroxacin, grepafloxacin, sparfloxacin, trovafloxacin, clinafloxacin, gatifloxacin, moxifloxacin, gemifloxacin, and sitafloxacin); rifamycins (e.g., rifampicin, rifabutin, rifapentine, and rifaximin); metronidazole; garenoxacin; ramoplanin; faropenem; polymyxin; tigecycline, AZD2563; and trimethoprim.

These antibiotics can be used in the dose ranges and formulations currently known and used for these agents. Different concentrations may be employed depending on the clinical condition of the subject, the goal of therapy (treatment or prophylaxis), the anticipated duration, and the severity of the *C. difficile* infection. Additional considerations in dose selection include the type of infection, age of the subject (e.g., pediatric, adult, or geriatric), general health, and comorbidity. Determining what concentrations to employ are within the skills of the pharmacist, medicinal chemist, or medical practitioner. Typical dosages and frequencies are provided, e.g., in the Merck Manual of Diagnosis & Therapy (17th Ed. MH Beers et al., Merck & Co.) and Physicians' Desk Reference 2003 (57th Ed. Medical Economics Staff et al., Medical Economics Co., 2002).

In one example rifalazil is administered in combination with vancomycin. Either the rifalazil or the vancomycin or both may be given daily (e.g., once, twice, three times, or four times daily) or less frequently (e.g., once every other day, once every three days, once or twice weekly, or monthly). Typical daily dosages for vancomycin range from 20 mg to 2 gm, preferably 125 mg to 2 gm, or 500 mg to 2 gm, but it may be administered in any higher tolerated amounts as necessary. Daily dosages of vancomycin can be can be distributed over one to four doses. Exemplary daily oral dosages include from 500 mg to 2 gm distributed over one to four doses for adult subjects and 40 mg/kg distributed over one to four doses for pediatric subjects. Intravenous administration can be given as a one-time bolus per 24-hour period, or for any subset of time over the 24-hour period (e.g., half an hour, one hour, two hours, four hours, up to 24 hours).

For combination therapy, the rifalazil and the additional antibiotic can be administered simultaneously or sequentially. For sequential administration, the rifalazil can be administered before, during, or after administration of the additional antibiotic, or any combination thereof. In one example, vancomycin is administered for five days and rifalazil is administered as a single dose on the sixth day. In another example, vancomycin and rifalazil are administered simultaneously on day one followed by administration of vancomycin for an additional six days. These examples are provided to illustrate two potential combinations for sequential therapy.

For combination therapy, the dosage and the frequency of administration of each component of the combination can be controlled independently. For example, one of the compounds (i.e., rifalazil or the additional antibiotic) may be administered three times per day, while the second compound may be administered once per day. The compounds may also be formulated together such that one administration delivers both compounds. In one particularly preferred embodiment, rifalazil is administered orally or intravenously, while the additional antibiotic is administered orally. In another preferred embodiment, both rifalazil and the additional antibiotic (e.g., vancomycin) are administered orally.

The following examples are shown to illustrate the present invention.

### Examples

### Example 1. Animal Models of C. difficile-associated disease

Optimal dosages and formulations of rifalazil alone, or in combination with a second drug compound, can be determined using standard animal models known in the art. One example of an animal model for *C. difficile* associated disease is the Golden Syrian hamster. To determine the optimal dosage regimen of rifalazil, Golden Syrian hamsters are injected subcutaneously with clindamycin phosphate (10mg/kg) followed, 24 hours later, by oral gavage with 10⁵ colony forming units (CFU) of *C. difficile*. Antibiotic treatment is then administered orally, intravenously, or intraperitoneally, either simultaneously or 24 hours after *C. difficile* administration. Animals are monitored for survival, weight variations, identification of *C. difficile* toxins in cecal content, and histologic damage to ceca as compared to animals treated with a prophylactic protocol using standard methods known in the art (see, for example, Anton P.M. et al., Abstract ID No. 102471, Publishing ID No. T1741, presented at the American Gastroenterological Association Meeting, May 17-22, 2003; Anton P.M. et al., Gastroenterology 124:A558, 2003).

### Example 2. Minimum inhibitory concentration of rifalazil (ABI1648) for 32 strains of C. difficile.

The minimum inhibitory concentration (MIC) of rifalazil was determined for 32 strains of *C. difficile*. All *C. difficile* isolates were collected in U.S. hospitals from 1987 to 1992. MICs were determined by agar dilution using NCCLS defined methodology (National Committee for Clinical Laboratory Standards, Methods for Antimicrobial Susceptibility Testing of Anaerobic Bacteria, Approved Standard M11-A5, Fifth Ed., 2001). Rifalazil and metronidazole were tested over an eleven doubling dilution concentration range.

Agar dilution testing panels were prepared on the day of testing. Agar containing vitamin K and hemin was melted and cooled to 50°C and 1 mL of blood supplement was added at this time. Prepared concentrations of each antimicrobial agent were added to the appropriate agar tube and mixed thoroughly. The agar mixture was poured into round petri dishes and allowed to solidify; plates were then placed in an incubator at 35-37°C for 30 minutes to allow for excess moisture on the agar surface to evaporate.

For agar dilution susceptibility testing, isolates frozen at -70°C were thawed and subcultured twice onto Brucella agar, with incubation at 35°C in 4-7% CO₂ for 48-72 hours following each subculture. A bacterial suspension was prepared in Brucella broth and adjusted to the density of a 0.5 McFarland standard. Agar plates with appropriate concentrations of antimicrobial agents were inoculated with 1-2 µl of bacterial suspension (10⁵ CFU per spot). Upon inoculation, plates were inverted and incubated at 35-37°C in an anaerobic environment. Plates were read after 48 hours of incubation and MICs were determined.

MICs for rifalazil were compared to MICs for metronidazole which were also determined for the 31 strains of *C. difficile*. These results are shown in Table 1.

**Table 1. MICs (µg/mL) for rifalazil and metronidazole on 31 strains of C. difficile.**

| **Sample #** | **Organism ID** | **Metronidazole** | **ABI1648** | **Identity** |
|---|---|---|---|---|
| **QC** | *B. frag* 25285 | 1 | 0.03* | NA |
| **QC** | *B. theta* 29741 | 2 | 0.03 | NA |
| **1** | *C. difficile* 18 | 0.5 | 0.001 | Different |
| **2** | *C. difficile* 22 | 0.5 | 0.001 | Different |
| **3** | *C. difficile* 28 | 0.5 | 0.002 | Different |
| **4** | *C. difficile* 40 | 0.5 | 0.001 | Different |
| **5** | *C. difficile* 44 | 0.5 | 0.001 | Different |
| **6** | *C. difficile* 50 | 0.5 | 0.002 | Different |
| **7** | *C. difficile* 60 | 0.5 | 0.002 | Not typable |
| **8** | *C. difficile* 70 | 0.25 | 0.001 | Not typable |
| **9** | *C. difficile* 80 | 0.5 | >0.5 | Different |
| **10** | *C. difficile* 101 | 0.5 | 0.004 | Different |
| **11** | *C. difficile* 107 | 0.5 | 0.001 | Different |
| **12** | *C. difficile* 113 | 0.5 | 0.004 | Different |
| **13** | *C. difficile* 116 | 0.25 | 0.002 | Not typable |
| **14** | *C. difficile* 143 | 0.5 | < =0.0005 | Different |
| **15** | *C. difficile* 146 | 0.5 | 0.001 | Different |
| **16** | *C. difficile* 154 | 0.25 | <=0.0005 | Not typable |
| **17** | *C. difficile* 166 | 0.5 | 0.001 | Different |
| **18** | *C. difficile* 199 | 0.25 | <=0.0005 | Different |
| **19** | *C. difficile* 65C | 0.5 | 0.002 | Not typable |
| **20** | *C. difficile* 5-5-03 | 0.5 | 0.002 | Not typable |
| **21** | *C. difficile* 6-5-03 | 0.5 | 0.002 | Different |
| **22** | *C. dilficile* 7-5-03 | 0.25 | 0.002 | Different |
| **23** | *C. difficile* 11-5-03 | 0.5 | 0.001 | Not typable |
| **24** | *C. difficile* 13-5-03 | 0.5 | <=0.0005 | Different |
| **25** | *C. difficile* 14-5-03 | 0.5 | 0.001 | Not typable |
| **26** | *C. difficile* 16-5-03 | 0.5 | 0.008 | Different |
| **27** | *C. difficile* 17-5-03 | 0.25 | 0.002 | Different |
| **28** | *C. difficile* 19-5-03 | 0.5 | 0.002 | Different |
| **29** | *C. difficile* 22-5-03 | 0.25 | 0.001 | Different |
| **30** | *C. difficile* 29-5-03 | 0.5 | >0.5 | Different |
| **31** | QC *C. diff ATCC* 43255 | 0.5 | 0.002 | Different |

| | | | | |
|---|---|---|---|---|
| *It should be noted that Fujii et al., (Antimicrob. Agents Chemother. 38:1118-1122, 1994) obtained a value of 1.0 for this organism, a value which differs from that described in Table 1. | | | | |

These data demonstrate that rifalazil was much more effective than metronidazole against almost all 31 strains of *C. difficile.* MICs for rifalazil were in the range of 0.0005 to 0.5 µg/mL while MICs for metronidazole were in the range of 0.25 to 0.5 µg/mL. Furthermore, identity analyses of these strains were performed, where possible, and indicate that the strains of *C. difficile* tested were not identical.

Strains 80 and 29-5-30 appeared to be less susceptible to metronidazole and rifalazil and therefore indicate the need for combination treatment described herein for the effective treatment of some strains of *C. difficile*.

## Claims

1. Use of rifalazil for the preparation of a pharmaceutical composition for treating an infection of *Clostridium difficile* or for preventing an infection of *Clostridium difficile* in a subject, wherein rifalazil is to be administered to said subject in an effective amount.

2. The use of claim 1, wherein said rifalazil is to be administered in an amount between 1 and 1000 mg/day, preferably between 1 and 100 mg/day, more preferably between 5 and 25 mg/day.

3. The use of claims 1 or 2, wherein said rifalazil is to be administered for one to fourteen days, preferably three to seven days.

4. The use of any one of claims 1-3, wherein said rifalazil is to be administered as a single dose.

5. The use of any one of claims 1-3, wherein said rifalazil is to be administered at an initial dose of between 5 and 100 mg, followed by subsequent doses of between 1 and 50 mg for three to seven days.

6. The use of any one of claims 1-5, wherein said infection of *Clostridium difficile* comprises a strain of *Clostridium difficile* that is resistant to one or more antibiotic selected from vancomycin; rifamycins, for example, rifampicin, rifabutin, rifapentine, rifaximin; and metronidazole.

7. The use of any one of claims 1-6, wherein said rifalazil is to be administered orally, intravenously, subcutaneously, or rectally.

8. The use of any one of claims 1-7, wherein said pharmaceutical composition is to be administered to said subject in combination with an agent that binds *Clostridium difficile* toxin A or toxin B.

9. The use of claims 1-8, wherein said pharmaceutical composition is to be administered to said subject in combination with a second antibiotic.

10. The use of claim 9, wherein said second antibiotic is metronidazole.

## Patentansprüche

1. Verwendung von Rifalazil für die Herstellung einer pharmazeutischen Zusammensetzung zum Behandeln einer Infektion von *Clostridium difficile* oder zum Verhindern einer Infektion von *Clostridium difficile* in einem Subjekt, wobei Rifalazil dem Subjekt in einer effektiven Menge zu verabreichen ist.

2. Verwendung nach Anspruch 1, wobei das Rifalazil in einer Menge zwischen 1 und 1000 mg/Tag, vorzugsweise zwischen 1 und 100 mg/Tag, bevorzugter zwischen 5 und 25 mg/Tag, zu verabreichen ist.

3. Verwendung nach Anspruch 1 oder 2, wobei das Rifalazil für einen bis vierzehn Tage, vorzugsweise drei bis sieben Tage, zu verabreichen ist.

4. Verwendung nach einem der Ansprüche 1 - 3, wobei das Rifalazil als eine einzelne Dosis zu verabreichen ist.

5. Verwendung nach einem der Ansprüche 1 - 3, wobei das Rifalazil in einer Anfangsdosis zwischen 5 und 100 mg, gefolgt von anschließenden Dosen zwischen 1 und 50 mg für drei bis sieben Tage zu verabreichen ist.

6. Verwendung nach einem der Ansprüche 1 - 5, wobei die Infektion von *Clostridium difficile* eine Kette von *Clostridium difficile* umfasst, die gegen ein oder mehrere Antibiotika, ausgewählt unter Vancomycin; Rifamycinen, zum Beispiel Rifampicin, Rifabutin, Rifapentin, Rifaximin; und Metronidazol, resistent ist.

7. Verwendung nach einem der Ansprüche 1 - 6, wobei das Rifalazil oral, intravenös, subkutan oder rektal zu verabreichen ist.

8. Verwendung nach einem der Ansprüche 1 - 7, wobei die pharmazeutische Zusammensetzung dem Subjekt in einer Kombination mit einem Mittel zu verabreichen ist, das *Clostridium difficile* -Toxin A oder -Toxin B bindet.

9. Verwendung nach den Ansprüchen 1 - 8, wobei die pharmazeutische Zusammensetzung dem Subjekt in Kombination mit einem zweiten Antibiotikum zu verabreichen ist.

10. Verwendung nach Anspruch 9, wobei das zweite Antibiotikum Metronidazol ist.

## Revendications

1. Utilisation du rifalazil pour la préparation d'une composition pharmaceutique pour traiter une infection de *Clostridium difficile* ou pour prévenir une infection de *Clostridium difficile* dans un sujet, le rifalazil devant être administré audit sujet dans une quantité efficace.

2. Utilisation selon la revendication 1, dans laquelle ledit rifalazil doit être administré dans une quantité entre 1 et 1 000 mg/jour, de préférence entre 1 et 1 00 mg/jour, de façon davantage préférée entre 5 et 25 mg/jour.

3. Utilisation selon l'une des revendications 1 ou 2, dans laquelle ledit rifalazil doit être administré pendant un à quatorze jours, de préférence trois à sept jours.

4. Utilisation selon l'une quelconque des revendications 1-3, dans laquelle ledit rifalazil doit être administré sous la forme d'une simple dose.

5. Utilisation selon l'une quelconque des revendications 1-3, dans laquelle ledit rifalazil doit être administré à une dose initiale d'entre 5 et 100 mg, en faisant suivre par des doses ultérieures d'entre 1 et 50 mg pendant trois à sept jours.

6. Utilisation selon l'une quelconque des revendications 1 à 5, dans laquelle ladite infection de *Clostridium difficile* comprend une souche de *Clostridium difficile* qui est résistante à un ou plusieurs antibiotiques choisis parmi la vancomycine ; les rifamycines, par exemple, la rifampicine, la rifabutine, la rifapentine, la rifaximine ; et le métronidazole.

7. Utilisation selon l'une quelconque des revendications 1-6, dans laquelle ledit rifalazil doit être administré par voie orale, par voie intraveineuse, par voie sous-cutanée ou par voie rectale.

8. Utilisation selon l'une quelconque des revendications 1-7, dans laquelle ladite composition pharmaceutique doit être administrée audit sujet en combinaison avec un agent qui lie la toxine A ou la toxine B de *Clostridium difficile.*

9. Utilisation selon l'une des revendications 1-8, dans laquelle ladite composition pharmaceutique doit être administrée audit sujet en combinaison avec un second antibiotique.

10. Utilisation selon la revendication 9, dans laquelle ledit second antibiotique est le métronidazole.
